# EUROPEAN PATENT APPLICATION

(11) **EP 2 700 404 A1**
(43) Date of publication of application: **26.02.2014**
(21) Application number: 13181580.5
(22) Date of filing: 23.08.2013
(51) Int. Cl.: A61K 31/575, A61K 45/06, A61P 35/00

(54) **Antcin derivatives in combination with an anti-cancer drug in treatment and/or prevention of tumors**

(30) Priority: 24.08.2012 US 201261692769 P
(71) Applicant: Taiwan Antitumor Biotech Co., Ltd., Taipei (TW)
(72) Inventor: Wu, Tzong-Zeng, Hualien 97401 (TW); Kao, I Kai, Hualien 97401 (TW)
(74) Representative: Letzelter, Felix Phillip

(57) **Abstract**

The invention surprisingly found that antcin or an ester derivative, salt or a composition thereof in combination with an anti-tumor drug provides unexpected efficacy in inhibiting tumor, while retaining normal cells at high viability. In addition, the amount of the anti-tumor drug in chemotherapy can be reduced by combinatorially using antcin or an ester derivative, salt or a composition thereof in view of the fact that they play a role in adjuvant therapy.

## Description

### Field of the Invention

This invention relates to a combination and a method of inhibiting and/or treating a tumor. In particular, the invention provides a combination of an anti-tumor drug and antcin or a derivative, salt or composition thereof in inhibiting and/or treating tumors.

### Background of the Invention

In spite of recent medical progress, cancer continues to be one of the most common and deadly diseases. The occurrence of cancer increases with aging over a life time ("lifetime risk"). Other risk factors are believed to include genetics, diet, and environmental exposure (e.g., to mutagenic chemicals, radiation, transforming viruses, etc.). Cancer is a term used to describe a wide variety of diseases that are each characterized by the uncontrolled growth of a particular type of cell. It begins in a tissue containing such a cell and, if the cancer has not spread to any additional tissues at the time of diagnosis, may be treated by, for example, surgery, radiation, or another type of localized therapy. However, when there is evidence that cancer has metastasized from its tissue of origin, different approaches to treatment are typically used. Indeed, because it is not possible to determine the extent of metastasis, systemic approaches to therapy are usually undertaken when any evidence of spread is detected. These approaches involve the administration of chemotherapeutic drugs that interfere with the growth of rapidly dividing cells, such as cancer cells.

For example, primary hepatocellular carcinoma (hereinafter may be referred to as "liver cancer") most often appears after progression of chronic hepatitis to cirrhosis. Many viral hepatitis and cirrhosis patients have been recognized, and, in recent years, incidence of liver cancer has been increasing. Liver cancer has been treated with, for example, hepatectomy, percutaneous local therapy (e.g., radiofrequency ablation therapy or ethanol injection therapy), transcatheter hepatic arterial embolization (TAE), continuous arterial infusion chemotherapy, or radiation therapy. Another example is breast cancer. A lobular carcinoma *in situ* and a ductal carcinoma *in situ* are breast cancers that have developed in the lobules and ducts, respectively, but have not spread to the fatty tissue surrounding the breast or to other areas of the body. An infiltrating (or invasive) lobular and a ductal carcinoma are cancers that have developed in the lobules and ducts, respectively, and have spread to either the breast's fatty tissue and/or other parts of the body. Other cancers of the breast include medullary carcinomas, colloid carcinomas, tubular carcinomas, and inflammatory breast cancer. Treatments available for breast cancer patients are surgery, immunotherapy, radiation therapy, chemotherapy, endocrine therapy, or a combination thereof. Several chemotherapeutic agents are in use in the treatment of cancer, including alkylating agents, antimetabolites antagonists, anticancer antibiotics, and plant-derived anticancer agents. However, most of the cytotoxic drugs used in cancer chemotherapy have narrow chemotherapeutic utility and serious side effects at high dosages.

Plants and mushrooms are a valuable resource for the discovery and development of novel, naturally derived agents to treat cancer. *Taiwanofungus camphoratus,* also called *Ganoderma comphoratum, Antrodia cinnamomea* or *Antrodia camphorate,* is known as niuchang-chih or niuchang-ku, and the host plant, niu-chang, is the Chinese common name for *Cinnamomum kanehirai,* which is an endangered species in Taiwan. Traditionally, *Taiwanofungus camphoratus* is used as a Chinese remedy for food, alcohol, and drug intoxication, diarrhea, abdominal pain, hypertension, skin itches, and liver cancer. Triterpenoids are the main components of *Taiwanofungus camphoratus.* Three triterpenoids, antcin A, antcin B and antcin C, are ergostane-type analogues and isolated from the fruiting bodies of *Taiwanofungus camphoratus.* Antcin A and C have potent immunomodulatory effects against reactive oxygen species. Yun-Chih Hsieh et al. further reports that antcin A, antcin C and methyl anticinate A can selectively inhibit proliferation of human cancer cells rather than normal cells (Yun-Chih Hsieh et al., "Methyl Antcinate A from Antrodia camphorate Induces Apoptosis in Human Liver Cancer Cells through Oxidant-Mediated Cofilin- and Bax-Triggered Mitochondrial Pathway," Chem. Res. Toxicol. 2010, 23, pp. 1256-1267).

However, there is a need for a relatively low toxicity and efficient method for treating and/or preventing tumors and inhibiting growth of tumors; in particular, a need that additionally ameliorates the toxicity generally associated with systemic chemotherapy is desired.

### Summary of the Invention

The invention provides a combination comprising an anti-tumor drug in combination with antcin or a salt or ester derivative or a composition thereof, wherein the antcin or a salt or ester derivative or a composition thereof is in an amount to reduce an amount of the anti-tumor drug and provide low cytotoxity to normal cells.

The invention also provides a method of inhibiting and/or treating tumor with selectivity to tumors in a subject, which comprises administering to a subject an effective amount of the combination of the invention.

### Brief Description of the Drawing

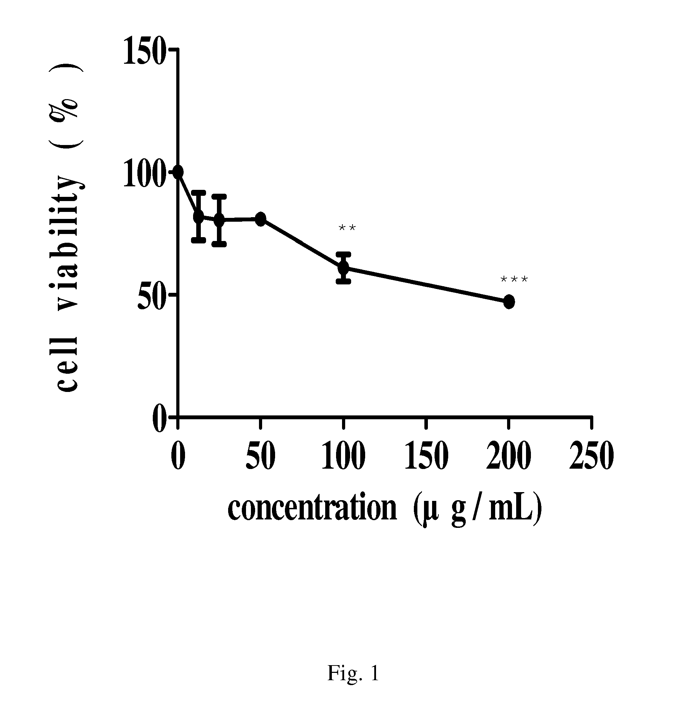

Figure 1 shows cell viability of HepG2 cells treated with 5FU in 50 *µ*g/ml, 100 *µ*g/ml, 150 *µ*g/ml and 200 *µ*g/ml, respectively. The "**" represents the p value in statistical significance testing as being less than 0.05 and the "***" represents the p value in statistical significance testing as being less than 0.001.

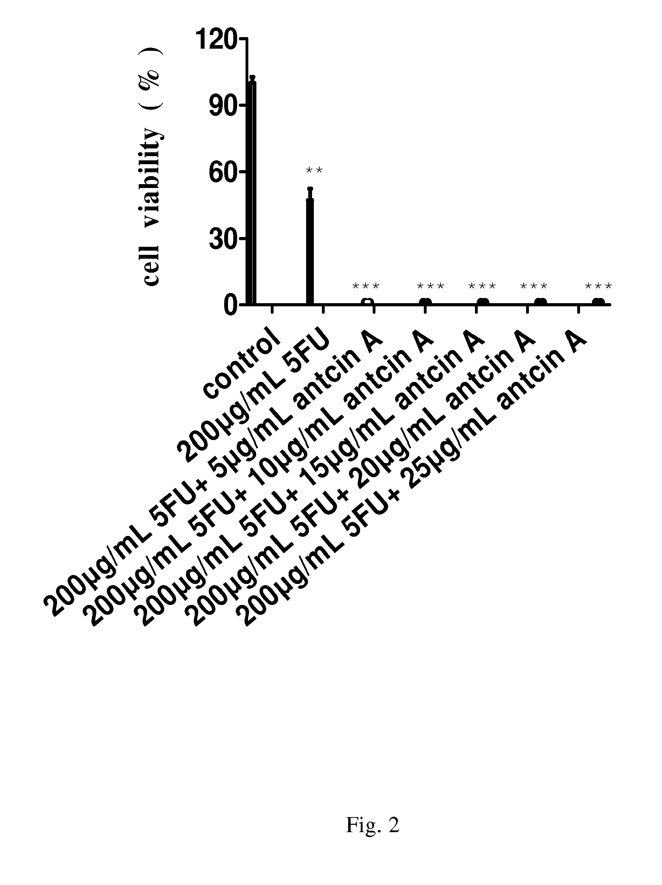

Figure 2 shows cell viability of HepG2 cells treated with 200 *µ*g/ml 5FU in combination with antcin A in 5 µg/ml, 10 µg/ml, 15 µg/ml, 20 µg/ml and 25 µg/ml, respectively. The "**" represents the p value in statistical significance testing as being less than 0.05 and the "***" represents the p value in statistical significance testing as being less than 0.001.

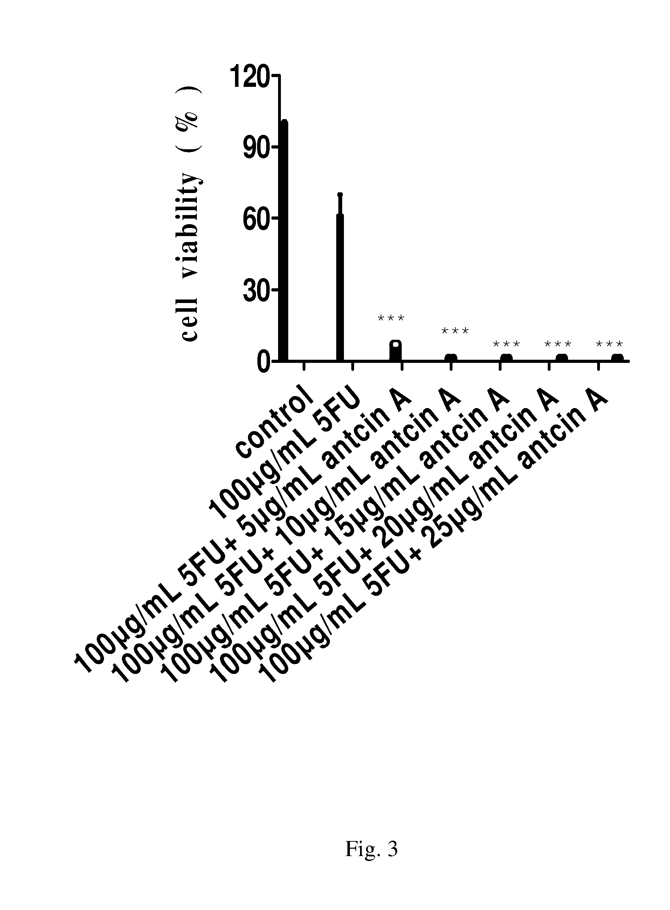

Figure 3 shows cell viabilities of HepG2 cells and CCD-966SK cells treated with 100 *µ*g/ml 5FU in combination with antcin A in 5 *µ*g/ml, 10 *µ*g/ml, 15 *µ*g/ml, 20 *µ*g/ml and 25 *µ*g/ml, respectively. The "***" represents the p value in statistical significance testing as being less than 0.001.

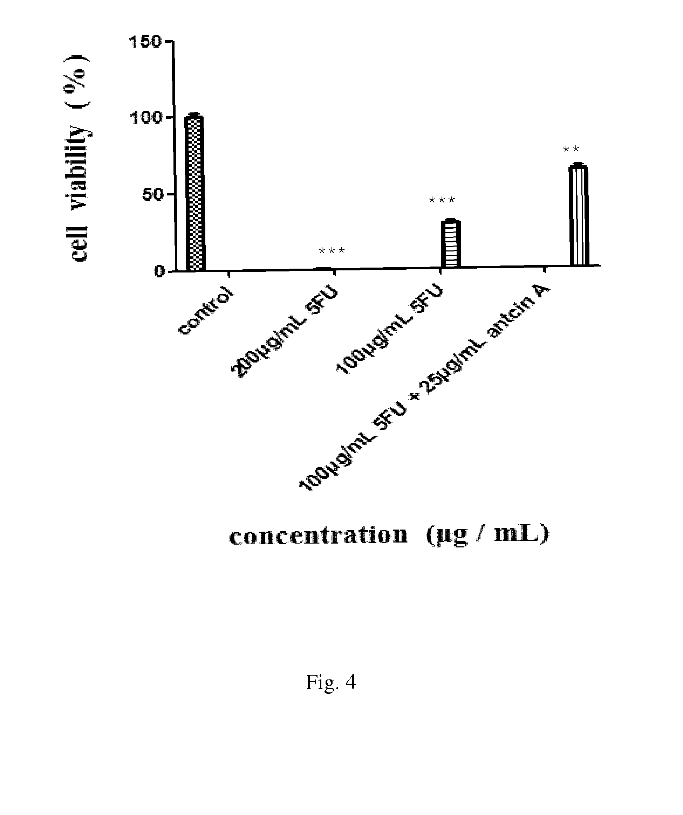

Figure 4 show cell viability of normal cell line CCD-966SK treated with 200 *µ*g/ml 5FU, 100 *µ*g/ml 5FU and 100 *µ*g/ml 5FU in combination with 25 *µ*g/ml antcin A. The "**" represents the p value in statistical significance testing as being less than 0.05 and the "***" represents the p value in statistical significance testing as being less than 0.001.

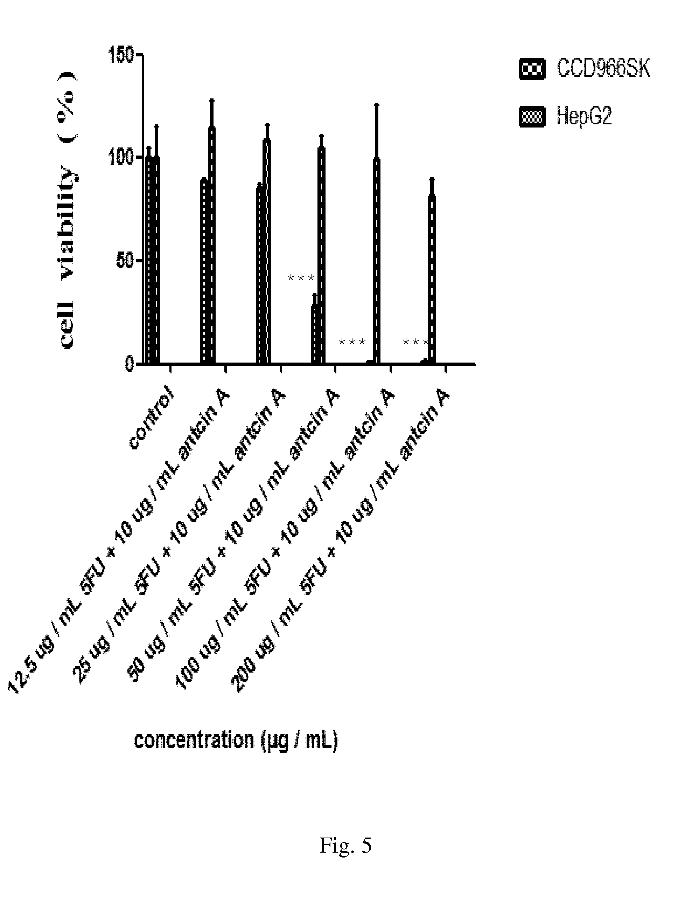

Figure 5 shows cell viabilities of HepG2 and CCD-966SK treated with -antcin A in 10 *µ*g/ml and 5-FUs at different concentrations. The "***" represents the p value in statistical significance testing as being less than 0.001.

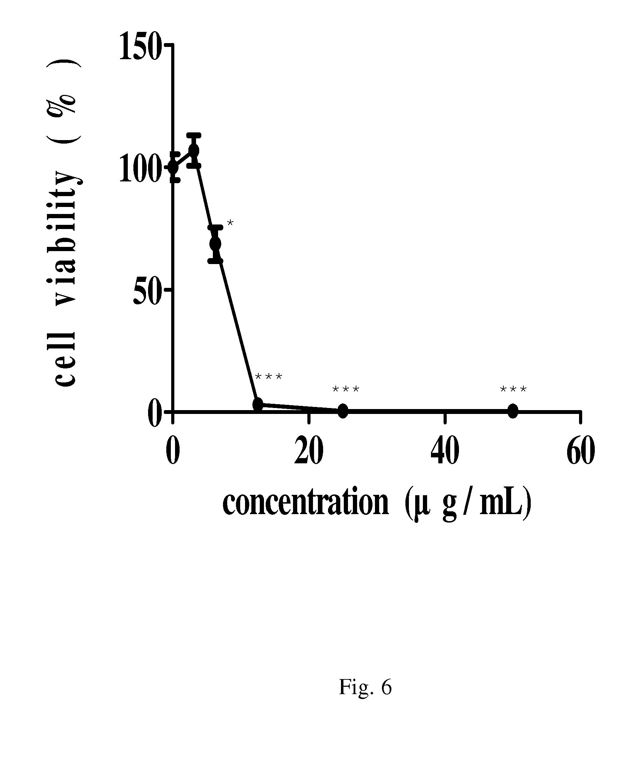

Figure 6 shows cell viability of HepG2 cells treated with tamoxifen. The "*" represents the p value in statistical significance testing as being less than 0.01; and the "***" represents the p value in statistical significance testing as being less than 0.001.

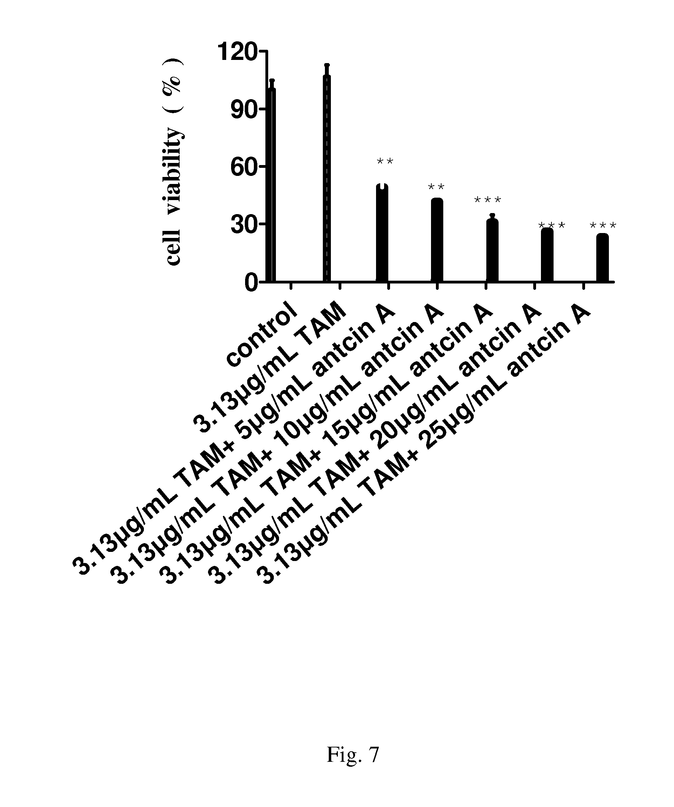

Figure 7 shows cell viability of HepG2 cells treated with 3.13 *µ*g/ml tamoxifen in combination with antcin A in 5 *µ*g/ml, 10 *µ*g/ml, 15 *µ*g/ml, 20 *µ*g/ml and 25 *µ*g/ml, respectively. The "**" represents the p value in statistical significance testing as being less than 0.05 and the "***" represents the p value in statistical significance testing as being less than 0.001.

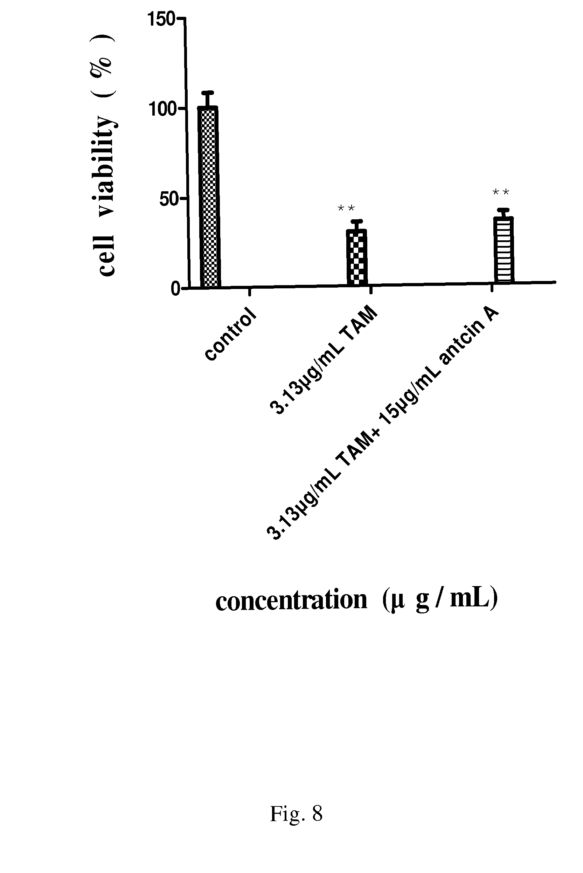

Figure 8 shows cell viability of normal cell line CCD-966SK treated with 3.13 *µ*g/ml TAM and 3.13 *µ*g/ml TAM in combination with 15 *µ*g/ml antcin A. The "**" represents the p value in statistical significance testing as being less than 0.05.

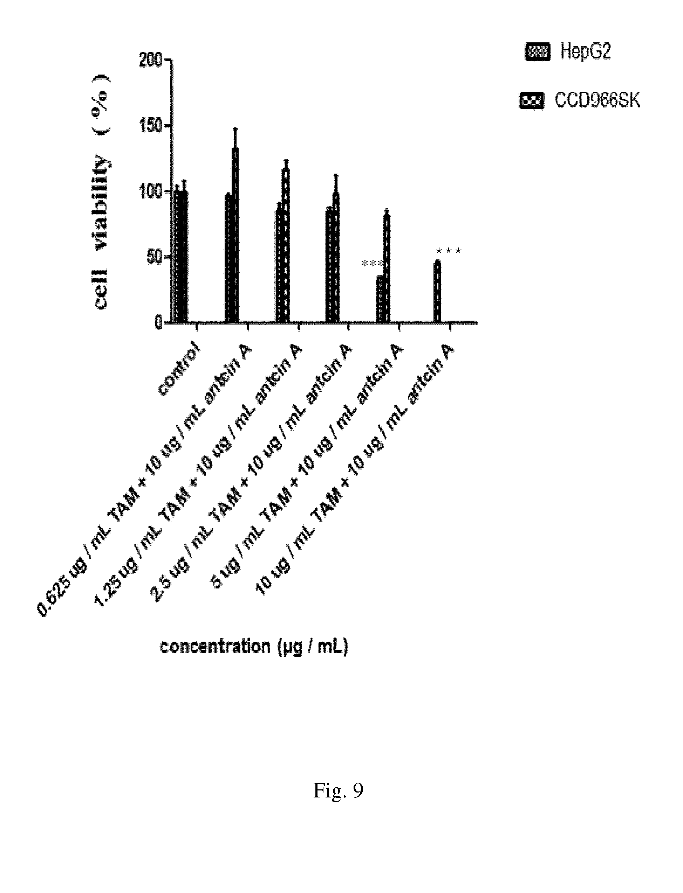

Figure 9 shows cell viabilities of HepG2 and CCD-966SK treated with antcin A in 10 *µ*g/ml and-TAM at different concentrations. The "***" represents the p value in statistical significance testing as being less than 0.001.

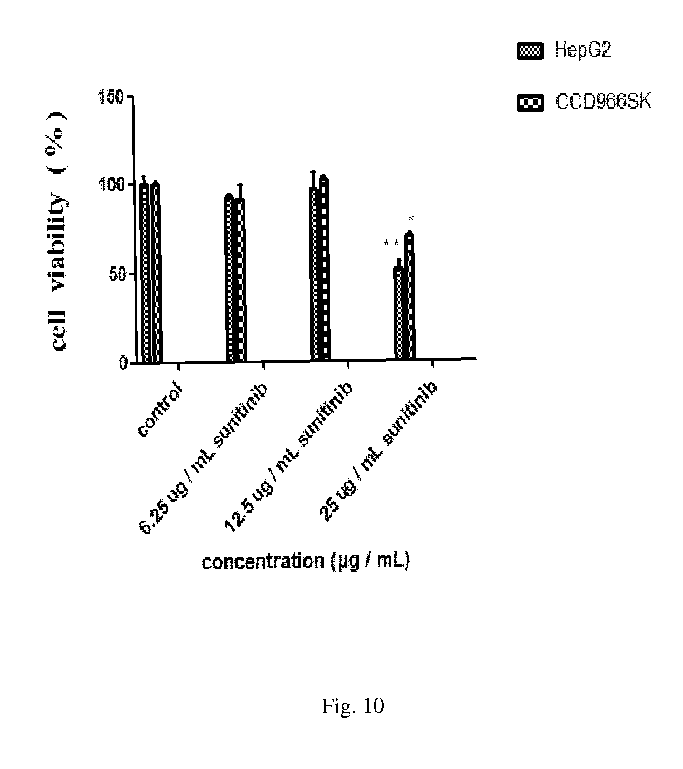

Figure 10 shows cell viabilities of HepG2 cells and CCD-966SK treated with sunitinib. The "*" represents the p value in statistical significance testing as being less than 0.01 and the "**" represents the p value in statistical significance testing as being less than 0.05.

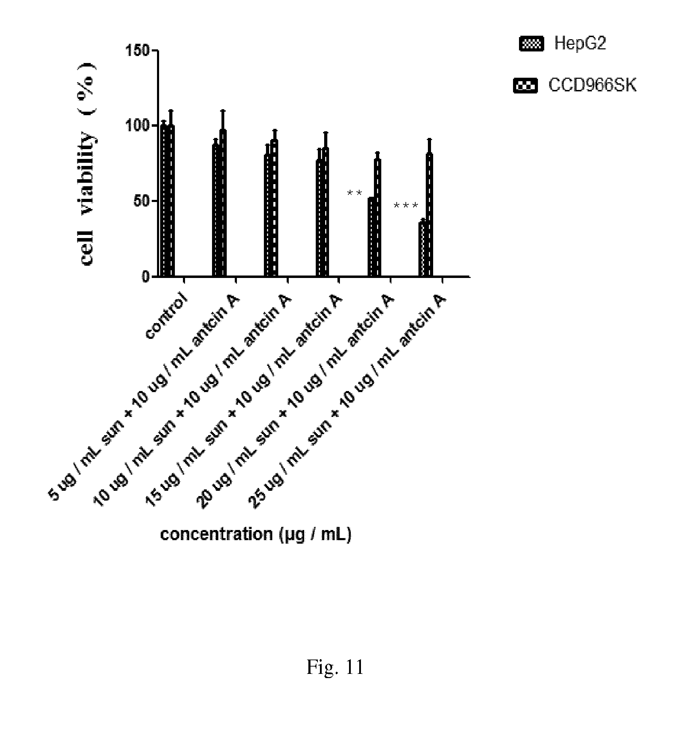

Figure 11 shows cell viabilities of HepG2 and CCD-966SK treated with antcin A in 10 *µ*g/ml and sunitinib at different concentrations. The "**" represents the p value in statistical significance testing as being less than 0.05 and the "***" represents the p value in statistical significance testing as being less than 0.001.

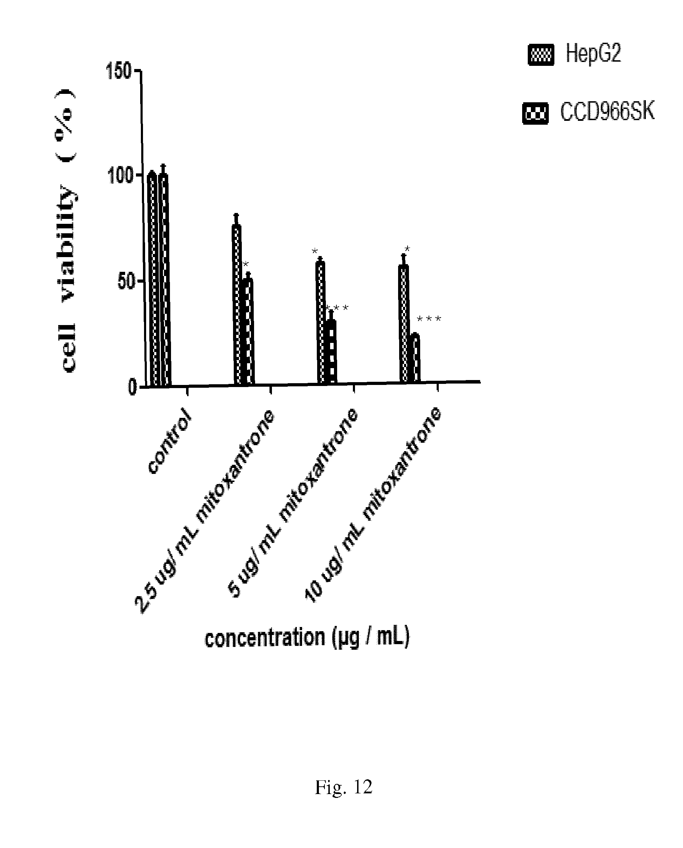

Figure 12 shows cell viabilities of HepG2 and CCD-966SK cells treated with mitoxantrone. The "*" represents the p value in statistical significance testing as being less than 0.01; the "**" represents the p value in statistical significance testing as being less than 0.05 and the "***" represents the p value in statistical significance testing as being less than 0.001.

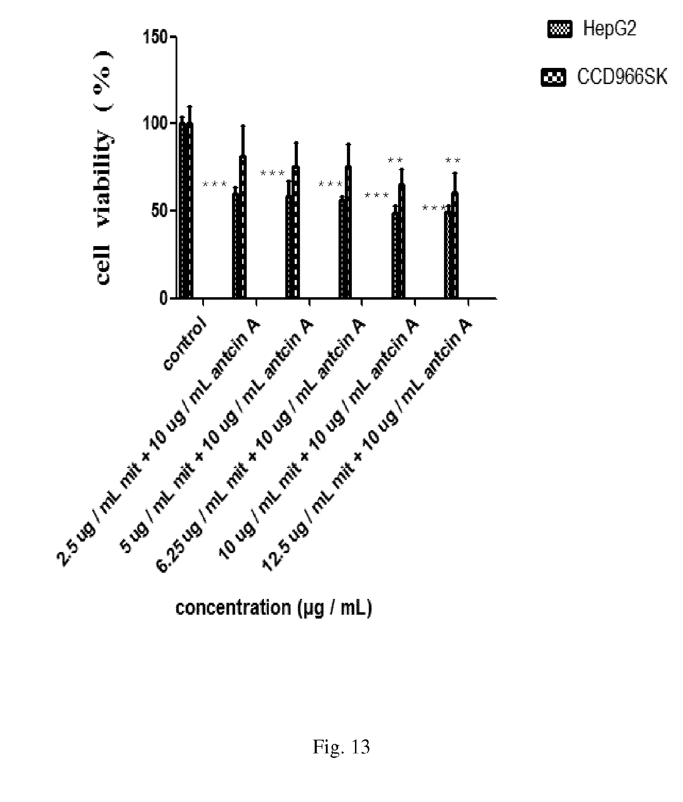

Figure 13 shows cell viabilities of HepG2 and CCD-966SK treated with antcin A in 10 *µ*g/ml and mitoxantrone at different concentrations. The "**" represents the p value in statistical significance testing as being less than 0.05 and the "***" represents the p value in statistical significance testing as being less than 0.001.

### Detailed Description of the Invention

The invention surprisingly found that an anti-tumor drug in combination with antcin or an ester derivative, salt or a composition thereof provides unexpected efficacy in inhibiting tumor cells, while retaining normal cells at high viability. In addition, the amount of the anti-tumor drug in chemotherapy can be reduced by combinatorially using antcin or an ester derivative, salt or a composition thereof in view of the fact that they play a role in adjuvant therapy. Furthermore, the combination of the invention comprising antcin or an ester derivative, salt or a composition thereof in combination with an anti-tumor drug can selectively inhibit tumor cells.

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The terms "a" and "an" refer to one or to more than one (i.e., to at least one) of the grammatical object of the article.

The term "treat," "treatment" or "treating" means reducing the frequency, extent, severity and/or duration with which symptoms of cancer are experienced by a patient.

The term "prevent," "prevention" or "preventing" means inhibition or the averting of symptoms associated with cancer.

The term "alleviate," "alleviation" or "alleviating" means reducing the severity of one or more symptoms of a disease, disorder or condition.

The term "salt" refers to those salts which retain the biological effectiveness and properties of the free bases and which are obtained by reaction with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid, malic acid, maleic acid, succinic acid, tartaric acid, citric acid, and the like.

The term "effective amount" means an amount of the combination of the invention effective to inhibit or treat and/or prevent cancer with low toxicity. For example, the effective amount of the combination of the invention may reduce the number of cancer cells; reduce the tumor size; inhibit (i.e., slow to some extent and preferably stop) cancer cell infiltration into peripheral organs; inhibit (i.e., slow at least to some extent and preferably stop) tumor metastasis; inhibit, to some extent, tumor growth; promote apoptosis; and/or relieve to some extent one or more of the symptoms associated with the disorder.

The term "subject" is understood to refer to animals, typically mammalian animals, such as primates (humans, apes, gibbons, chimpanzees, orangutans, macaques), domestic animals (dogs and cats), farm animals (horses, cattle, goats, sheep, pigs) and experimental animals (mouse, rat, rabbit, guinea pig). Subjects include animal disease models (e.g., tumor-prone mice, tumor-bearing mice, or mice receiving xenograft tumors).

The term "tumor" is used herein to include, but not limited to, tumors originating in the breast, prostate, colon, lung, pancreas, liver, stomach, bladder, or reproductive tract (cervix, ovaries, endometrium etc.), brain, and bone marrow; melanoma; or lymphoma.

The term "inhibiting tumor cell growth" is used herein to mean one or more of slowing the growth of the tumor cells, halting growth of the tumor cells, causing reduction or regression of the tumor cells, inhibiting tumor invasion, causing tumor cell death, and causing reduction or regression of metastases.

In one aspect, the invention provides a combination comprising an anti-tumor drug in combination with antcin or a salt or ester derivative or a composition thereof, wherein the antcin or a salt or ester derivative or a composition thereof is in an amount to reduce an amount of the anti-tumor drug and provide low cytotoxity to normal cells.

*Taiwanofungus camphoratus* ethanol extract and antcins isolated therefrom or derivatives thereof can selectively inhibit tumor cells by inducing their apoptosis but exhibit with low toxicity to normal cells, whereby the *Taiwanofungus camphoratus* ethanol extract and antcins isolated therefrom or derivatives thereof can be used as agents against tumors or used in chemotherapy.

Antcins are ergostane-type triterpenoids isolated from the fruiting bodies of *Taiwanofungus camphoratus,* having the following formula: 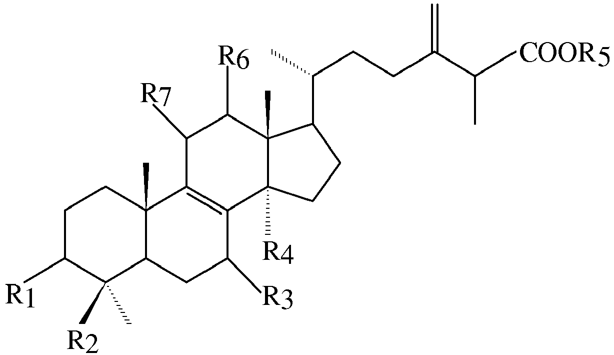 wherein R₁ is =O or OH; R₂ is H or OH; R₃ is H, =O, OH or O-acetyl; R₄ is H or OH; R₅ is H; R₆ is H or OH; and R₇ is =O or OH.

Examples of antcins include antcin A (4α-methylergosta-8,24(28)-diene-3,11-dione-26-oic acid), antcin B (2-methyl-3-methylidene-6-[(4S)-4,10,13-trimethyl-3,7,11-trioxo-1,2,4,5,6,12,14,15,16,17-decahydrocyclopenta[a]phenanthren-17-yl]heptanoic acid), antcin C (4amethylergosta- 8,24(28)-dien-3,11 -trion-7P-26-oic acid), antcin D (4,14-hydroxy-4α-methyl-3,7,11-trioxo-ergost-8,24(28)-dien-26-oic acid), antcin E (5,3,11-dioxo-4α- methylergost-8,14,24(28)-trine-26-oic acid), antcin F (6,3,11-dioxo-7β-hydroxy-4a- methylergost-8,14,24(28)-trine-26-oic acid), antcin H (3α,12a-dihydroxy-4a-methylergost-8,24(28)-dien-7,11 -dion-26-oic acid) and antcin K (4,7-dimethethoxy-5-methyl-1,3-benzodioxole). The chemical formula of antcins A-K are as follows:

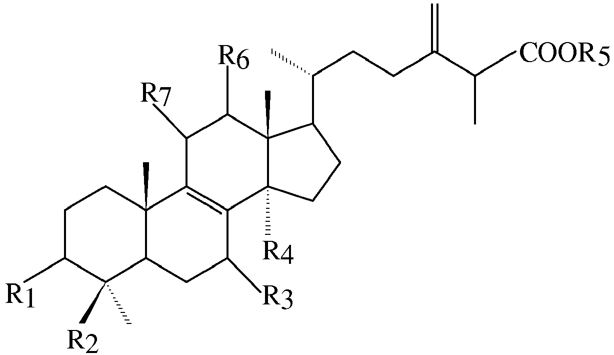

| Compound | R₁ | R₂ | R₃ | R₄ | R₅ | R₆ | R₇ |
|---|---|---|---|---|---|---|---|
| Antcin A | =O | H | H | H | H | H | =O |
| Antcin B | =O | H | =O | H | H | H | =O |
| Antcin C | =O | H | -OH | H | H | H | =O |
| Antcin D | =O | H | =O | H | H | H | =O |
| Antcin E | =O | H | H | -- | H | H | =O |
| Antcin F | =O | H | -OH | -- | H | H | =O |
| Antcin G | =O | H | -OAc | H | H | H | =O |
| Antcin H | -OH | H | =O | H | H | -OH | =O |
| Antcin I | -OH | H | =O | H | H | H | =O |
| Antcin J | =O | H | =O | -OH | H | H | -OH |
| Antcin K | -OH | -OH | -OH | H | H | H | =O |

Antcin A, 4α-methylergosta-8,24(28)-diene-3,11-dione-26-oic acid, is one preferred embodiment used in the combination of the invention and has the structural formula as follows:

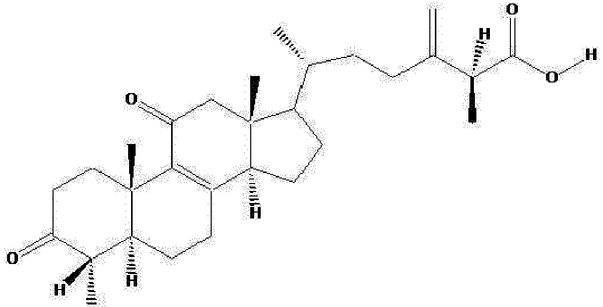

The salt of antcin includes base addition. Antcins of the invention which are acidic can form salts, including pharmaceutically acceptable salts, with bases such as alkali metal hydroxides, e.g. sodium and potassium hydroxides; alkaline earth metal hydroxides e.g. calcium, barium and magnesium hydroxides; with organic bases e.g. N-methyl-D-glucamine, choline tris(hydroxymethyl)amino-methane, L-arginine, L-lysine, N-ethyl piperidine, dibenzylamine and the like. Specific salts with bases include the benzathine, calcium, diolamine, meglumine, olamine, potassium, procaine, sodium, tromethamine and zinc salts. In one embodiment, the antcin ester derivatives includes, but not limited to, alkyl antcin, alkenyl antcin, alkynyl antcin. Preferably, the antcin ester is C₁₋₈ alkyl (such as methyl, ethyl, propyl and butyl etc.) antcinate A, C₁₋₈ alkyl (such as methyl, ethyl, propyl and butyl etc.) antcinate B, C₁₋₈ alkyl (such as methyl, ethyl, propyl and butyl etc.) antcinate C, C₁₋₈ alkyl (such as methyl, ethyl, propyl and butyl etc.) antcinate D, C₁₋₈ alkyl (such as methyl, ethyl, propyl and butyl etc.) antcinate E, C₁₋₈ alkyl (such as methyl, ethyl, propyl and butyl etc.) antcinate F, C₁₋₈ alkyl (such as methyl, ethyl, propyl and butyl etc.) antcinate H and C₁₋₈ alkyl (such as methyl, ethyl, propyl and butyl etc.) antcinate K. Preferably, the antcin ester is methyl antcinate A, methyl antcinate B, methyl antcinate C, methyl antcinate G, methyl antcinate H, ethyl antcinate A or ethyl antcinate B; the chemical formula thereof are as follows:

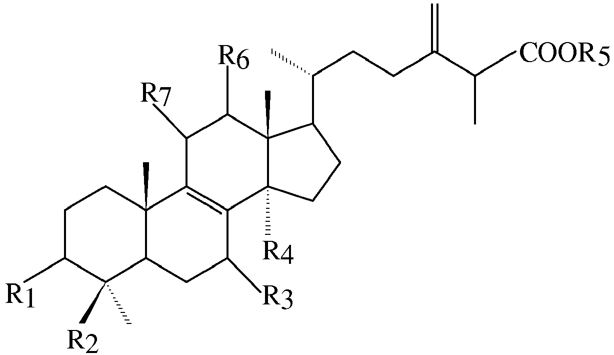

| Compound | R₁ | R₂ | R₃ | R₄ | R₅ | R₆ | R₇ |
|---|---|---|---|---|---|---|---|
| Methyl antcinate A | =O | H | H | H | CH₃ | H | =O |
| Methyl antcinate B | =O | H | =O | H | CH₃ | H | =O |
| Methyl antcinate C | =O | H | -OH | H | CH₃ | H | =O |
| Methyl antcinate G | =O | H | -OAc | H | CH₃ | H | =O |
| Methyl antcinate H | -OH | H | =O | H | CH₃ | -OH | =O |
| Ethyl antcinate A | =O | H | H | H | CH₂CH₃ | H | =O |
| Ethyl antcinate B | -OH | H | =O | H | CH₂CH₃ | -OH | =O |

In another embodiment, the composition of antcins is an extract of *Taiwanofungus camphoratus* containing antcin. Antcins or the derivatives thereof are included in an extract of *Taiwanofungus camphoratus.* One of the preferred embodiments is the low polar fraction of *Taiwanofungus camphoratus* ethanol extract. According to the invention, the low polar fraction of *Taiwanofungus camphoratus* ethanol extract can be obtained by extracting *Taiwanofungus camphoratus* with n-haxane eluting the resulting ethanol extract with silica gel chromatography using a mixture of methanol, and ethyl acetate in various ratios as a mobile phase and collecting the low polar fraction.

According to one embodiment of the invention, *Taiwanofungus camphoratus* can be in a powder or solution form. According to the invention, the low polar fraction can be further purified by high performance liquid chromatography (HPLC), preparative HPLC or half-preparative HPLC to obtain a fraction with retention time at 23-35 minutes. Preferably, the retention time is at around 24-33 minutes, around 27-33 minutes or around 28-33, more preferably, the retention time is at around 28-29 minutes. According to a more preferred embodiment of the invention, the resulting fraction is further purified to obtain antcins.

According to the invention, the anti-tumor drugs include, but are not limited to: a) drugs with antimitotic effects, especially those which target cytoskeletal elements, including microtubule modulators such as taxane drugs (such as taxol, paclitaxel, docetaxel), podophylotoxins or vinca alkaloids (vincristine, vinblastine); b) antimetabolite drugs (such as 5-fluorouracil (5FU), cytarabine, gemcitabine, purine analogues such as pentostatin, methotrexate); c) alkylating agents or nitrogen mustards (such as platinum drugs (cisplatin, carboplatin, oxaliplatin, paraplidineatin), nitrosoureas, cyclophosphamide or ifosfamide); d) drugs which target DNA such as the antracycline drugs adriamycin, doxorubicin, pharmorubicin or epirubicin; e) drugs with target topoisomerases such as camptothecin, etoposide, mitoxantrone, doxorubicin; f) hormones or hormone agonists or antagonists such as estrogens, antiestrogens (tamoxifen and related compounds) and androgens, flutamide, leuprorelin, goserelin, cyproterone or octreotide; g) drugs which target signal transduction in tumour cells including antibody derivatives such as herceptin; h) tyrosine kinase inhibitors such as imatinib, gefitinib and erlotinib and sunitinib, i) drugs potentially affecting metastasis of tumours such as matrix metalloproteinase inhibitors; j) gene therapy and antisense agents; and k) antibody therapeutics. Preferably, the anti-cancer drug is a drug with antimitotic effect, an antimitotic drug, an antimetabolite drug, an alkylating agent or nitrogen mustard, a drug which target DNA, a drug with target topoisomerase or a hormone antagonist. More preferably, the drug with antimitotic effect is taxol, paclitaxel, taxotere, docetaxel, podophylotoxins, vincristine or vinblastine; the antimetabolite drug is 5-fluorouracil (5FU), cytarabine, gemcitabine, pentostatin or methotrexate; the alkylating agent or nitrogen mustard is cis-platin, carbonplatin, oxaliplatin, paraplidineatin, nitrosoureas, cyclophosphamide or ifosphamide; the drug which target DNA is adriamycin, doxorubicin, pharmorubicin or epirubicin; the drug with target topoisomerases is camptothecin, etoposide, mitoxantrone or doxorubicin; the hormone or hormone agonist or antagonist is estrogen, tamoxifen, androgens, flutamide, leuprorelin, goserelin, cyprotrone or octreotide; the drug which target signal transduction in tumour cells is herceptin; and the tyrosine kinase inhibitor is imatinib, gefitinib and erlotinib or sunitinib. More preferably, the drug with antimitotic effects is paclitaxel, taxotere or docetaxel; the antimetabolite drug is 5FU; the alkylating agent or nitrogen mustard is cis-platin, carbonplatin or cyclophosphamide; the drug which target DNA is doxorubicin; the drug with target topoisomerases is camptothecin, mitoxantrone or doxorubicin; the antiestrogen is tamoxifen; and the tyrosine kinase inhibitor is sunitinib. Most preferably, the drug with antimitotic effects is paclitaxel or docetaxel; the antimetabolite drug is 5FU; the alkylating agent or nitrogen mustard is cis-platin or carbonplatin; the drug which target DNA is doxorubicin; the drug with target topoisomerases is mitoxantrone; the antiestrogen is tamoxifen; and the tyrosine kinase inhibitor is sunitinib.

In one embodiment, the invention provides a combination comprising an antimitotic drug, an antimetabolite drug, an alkyling agent, a nitrogen mustard, a drug which target DNA, a topoisomerase inhibitor, a hormone antagonist or a tyrosine kinase inhibitor in combination with antcin or a salt or ester derivative or a composition thereof. Preferably, the combination comprises paclitaxel, taxotere, docetaxel, 5FU, cis-platin, carbonplatin, cyclophosphamide, doxorubicin, camptothecin, mitoxantrone, tamoxifen, or sunitinib in combination with antcin or a salt or ester derivative or a composition thereof. In another embodiment, the invention provides a combination comprising 5-FU, tamoxifen, mitoxantrone or sunitinib in combination with antcin or a salt or ester derivative or a composition thereof.

In some embodiments, the antcin or a salt or ester derivative or a composition thereof is in an amount to reduce an amount of the anti-tumor drug and provide low cytotoxity to normal cells. Preferably, the antcin or a salt or ester derivative or a composition thereof in combination with an anti-tumor drug are in the amounts ranging from about 0.5% (w/w) to about 95% (w/w) and about 99.5% (w/w) to about 5% (w/w) respectively, about 2% (w/w) to about 90% (w/w) and about 98% (w/w) to about 10% (w/w) respectively, about 4% (w/w) to about 90% (w/w) and about 96% (w/w) to about 10% (w/w) respectively, about 2% (w/w) to about 85% (w/w) and about 98% (w/w) to about 15% (w/w) respectively, about 2% (w/w) to about 25% (w/w) and about 98% (w/w) to about 75% (w/w) respectively, about 55% (w/w) to about 90% (w/w) and about 45% (w/w) to about 10% (w/w) respectively, about 10% (w/w) to about 80% (w/w) and about 90% (w/w) to about 20% (w/w) respectively, about 15% (w/w) to about 70% (w/w) and about 85% (w/w) to about 30% (w/w) respectively, about 20% (w/w) to about 80% (w/w) and about 80% (w/w) to about 20% (w/w) respectively, about 20% (w/w) to about 70% (w/w) and about 80% (w/w) to about 30% (w/w) respectively, about 20% (w/w) to about 60% (w/w) and about 80% (w/w) to about 40% (w/w) respectively, about 20% (w/w) to about 50% (w/w) and about 80% (w/w) to about 50% (w/w) respectively, about 30% (w/w) to about 80% (w/w) and about 70% (w/w) to about 20% (w/w) respectively, about 30% (w/w) to about 70% (w/w) and about 70% (w/w) to about 30% (w/w) respectively, about 30% (w/w) to about 60% (w/w) and about 70% (w/w) to about 40% (w/w) respectively, about 40% (w/w) to about 80% (w/w) and about 60% (w/w) to about 20% (w/w) respectively, about 40% (w/w) to about 70% (w/w) and about 60% (w/w) to about 30% (w/w) respectively, and about 50% (w/w) to about 80% (w/w) and about 50% (w/w) to about 20% (w/w) respectively.

Pharmaceutical formulations that comprise the combination of the invention will typically comprise one or more carriers or excipients and optionally other therapeutic ingredients. The carrier(s) will generally be "acceptable" in the sense of being compatible with the other ingredients of the formulation and physiologically innocuous to the recipient thereof. Such carriers or excipients are known, e.g., fillers, lubricants, binders and various liquid excipients for liquid formulations. Suitable carriers include those disclosed in the references cited herein.

Preferably the combination of this invention are in unit dosage forms such as tablets, pills, capsules, powders, granules, sterile parenteral solutions or suspensions, metered aerosol or liquid sprays, drops, ampoules, auto-injector devices or suppositories; for oral, parenteral, intranasal, sublingual or rectal administration, or for administration by inhalation or insufflation. Alternatively, the combinations may be presented in a form suitable for once-weekly or once-monthly administration. An erodible polymer containing the active ingredient may be envisaged. For preparing solid combinations such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other diluents, e.g. water, to form a solid preformulation combination containing a homogeneous mixture of the compound of the present invention, or a salt, derivative or composition thereof. When referring to these preformulation combinations as homogeneous, it is meant that the ingredient is dispersed evenly throughout the combination so that the combination may be readily subdivided into equally effective unit dosage forms such as tablets, pills and capsules. The tablets or pills of the novel combination can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable formulations include aqueous or oily solutions of the combination of the invention. Formulations suitable for parenteral delivery of the active ingredient include aqueous and non-aqueous compositions where the active ingredient is dissolved or suspended in solution. Formulations suitable for parenteral administration include aqueous and non-aqueous sterile injection solutions which may contain anti-oxidants, buffers, bacteriostats or solutes that render the formulation isotonic with the blood of the intended recipient. Other parenteral formulations may comprise aqueous and non-aqueous sterile suspensions which may include suspending agents and thickening agents.

Formulations of the invention suitable for oral administration are prepared as discrete units such as capsules, cachets, gums or tablets each containing a predetermined amount of the low polar fraction of *Taiwanofungus camphoratus* ethanol extract or antcin A or its pharmaceutically salt; as a powder or granules; as solution or a suspension in an aqueous liquid or a non-aqueous liquid; or as an oil-in-water liquid emulsion or a water-in-oil liquid emulsion.

Formulations for rectal administration may be presented as a suppository with a suitable base.

Formulations suitable for intrapulmonary or nasal administration will have a particle size, for example, in the range of 0.01 to 200 microns (including particle sizes in a range between 0.01 and 500 microns in increments of 0.1 microns such as 0.1, 0.2, 0.3, 0.4, 0.5, 1, 2, 5, 30 microns, 35 microns, etc.), which is administered by inhalation through the nasal passage or by inhalation through the mouth so as to reach the various bronchi or alveolar sacs. Formulations suitable for aerosol or dry powder administration may be prepared according to conventional methods and may be delivered with other therapeutic agents.

Formulations suitable for vaginal administration may be presented as pessaries, tampons, creams, gels, pastes, foams or spray formulations containing in addition to the active ingredient such carriers as are known in the art to be appropriate.

Formulations suitable for transdermal administration may be presented as transdermal patches. The transdermal patch provides a base line or steady state nicotine level to the patient. The total amount of the low polar fraction of *Taiwanofungus camphoratus* ethanol extract or antcin A or its pharmaceutically salt released by the patch during the period of use will vary depending on the user's body size, history of exposure to nicotine, and response to treatment. The size of the patch will vary according to the amount of nicotine to be delivered.

Formulations comprising the combination of the invention are presented in unit-dose or multi-dose containers, for example sealed ampoules and vials, and may be stored in a freezedried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily subdose, as described herein.

It should be understood that in addition to the combination of the invention. particularly mentioned above the formulations of this invention may include other agents conventional in the art having regard to the type of formulation in question, for example those suitable for oral administration may include flavoring or coloring agents.

The combination of the invention may be used to provide controlled release pharmaceutical formulations in which the release of the combination of the invention. is controlled and regulated to allow less frequent dosing or to improve the pharmacokinetic or toxicity profile of the combination of the invention.

The formulations include those suitable for any of the foregoing administration routes. The formulations may conveniently be presented in unit dosage form and may be prepared by any of the methods well known in the art of pharmacy. Such methods include the step of bringing into association the active ingredient with the carrier which constitutes one or more accessory ingredients or excipients. In general, the formulations are prepared by uniformly and intimately bringing into association the active ingredient with liquid carriers or finely divided solid carriers or both, and then, if necessary, shaping the product. For example, a tablet is made by compression or molding, optionally with one or more accessory ingredients. Compressed tablets may be prepared by compressing in a suitable machine the combination of the invention in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

In another aspect, the invention provides a method of inhibiting and/or treating tumor cells with low toxicity, which comprises administering to a subject an effective amount of the combination of the invention comprising antcin or a salt or ester derivative or a composition thereof in combination with an effective amount of an anti-tumor drug. According to one embodiment of the invention, the antcin or a salt or ester derivative or a composition thereof and the anti-tumor drug can be administered separately, concurrently or sequently.

According to the invention, antcin or a salt or ester derivative or a composition thereof in combination with an anti-tumor drug are useful to inhibit tumor cells with low toxicity and selectivity. Particularly, the combination of the invention is cytotoxic to tumor cells but not to normal cells. The amount of anti-tumor drug can be decreased when combinatorially using antcin or a salt or ester derivative or a composition thereof. According to the invention, the tumor is myeloid lymphomas, hepatocellular carcinoma, hepatoblastoma, rhabdomyosarcoma, esophageal carcinoma, thyroid carcinoma, ganglioblastoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leimyosarcoma, rhabdotheliosarcoma, colon carcinoma, pancreatic cancer, liver cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, hematoma, bile duct carcinoma, melanoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependynoma, pinealoma, hemangioblastoma, retinoblastoma, leukemia (e.g. acute lymphocytic leukemia), acute myelocytic leukemia (myelolastic, promyelocytic, myelomonocytic, monocytic and erythroleukemia), chronic leukemia (chronic myelocytic (granulocytic) leukemia and chronic lymphocytic leukemia), polycythemia vera, lymphoma (Hodgkin's disease and non-Hodgkin's diseases), multiple myeloma, Waldenstrom's macroglobulinemia, rectal carcinoma, head and neck cancer, brain cancer, cancers of unknown primary site, cancers of the peripheral nervous system, cancers of the central nervous system, acoustic neuroma, oligodendroglioma, meningioma, neuroblastoma, heavy chain disease, metastases, and any disease or disorder characterized by uncontrolled or abnormal cell growth. Preferably, the tumor is lever tumors or breast tumors.

In one of embodiment, the tumor is liver cancer. The liver cancer includes hepatocellular carcinoma (HCC), hemangioendotheliomas, sarcoma, hepatoblastoma or bile duct cancer (cholangiocarcinoma). The combination of the invention may optionally be used in combination therapies to treat any of these diseases or conditions. The combination therapies include the combination of the invention combined with surgery, radiation therapy, chemotherapy, cytotoxic agents or cytostatic agents, including any of the therapies or treatments disclosed herein or in any of the references cited herein. Preferably, the combination of the invention can be used in combination with chemotherapy. Alternatively, the combination of the invention can be used as chemotherapeutic agent or an adjunctive chemotherapeutic agent.

An effective dose of the combination of the invention for use in therapeutic applications, will depend to a certain extent at least on factors such as the status of the condition being treated, whether the combination of the invention is being used prophylactically (lower doses) or the severity of the malignancy and the method of delivery. These factors will be determined by the clinician using conventional dose escalation studies.

Any suitable route of administration may be employed for providing the patient with an effective dosage of the combination of the invention. For example, oral, rectal, parenteral, transdermal, transmucosal, subcutaneous, intramuscular, intrathecal, intrapulmonary, nasal or vaginal and the like may be employed as appropriate.

The combination of the invention can effectively inhibit tumor cells but not normal cells. Therefore, the combination of the invention can selectively inhibit tumor cells with low toxicity. Moreover, by combinatorially using antcin or an ester derivative, salt or a composition thereof, the amounts of anti-tumor drug can be decreased, so the side effects caused by the anti-tumor are reduced.

### Examples

### Example 1 Cell Viability Assay (MTT assay) for Combination of antcin A and 5-fluorouracil (5FU)

2 x 10⁴ HepG2 cells were seeded to each well of 96-well plate and incubated with DMEM medium containing 10% FBS and 1% antibiotic-antimycotic solution. The medium was removed and then 6.25µg/ml 5-fluorouracil (5FU) as control, 200 µg/ml 5FU+25µg/ml antcin A, 200 µg/ml 5FU+20µg/ml antcin A, 200 µg/ml 5FU+15µg/ml antcin A, 200 µg/ml 5FU+10µg/ml antcin A, 200 µg/ml 5FU+5µg/ml antcin A, 100 µg/ml 5FU+25µg/ml antcin A, 100 µg/ml 5FU+20µg/ml antcin A, 100 µg/ml 5FU+15µg/ml antcin A, 100 µg/ml 5FU+10µg/ml antcin A and 100 µg/ml 5FU+5µg/ml antcin A were added to the wells. After 24 hours, the agents were removed and 100µl of 0.5% mg/ml 3-(4,5-dimethyl-thiazol-2-yl)-2,5-diphenyltetrazolium bromide (MTT) was added to each well for MTT assay. After incubated under dark at 37°C for 2 hours, the solution was removed and 100 µl DMSO was added to each well and placed for 10 minutes under dark. The solution was taken from each well and then subjected to ELISA reader to detect absorbance at 570 nm wavelength. The cell viability percent was calculated by using absorbance of the control as 100%.

As shown in Fig. 1, IC₅₀ of HepG2 cells treated with 5FU is 200 *µ*g/ml and the cells even in a high concentration of 5FU cannot be completely killed. However, the cells treated with 200 *µ*g/ml 5FU in combination with antcin A in 5 *µ*g/ml, 10 *µ*g/ml, 15 *µ*g/ml, 20 *µ*g/ml and 25 *µ*g/ml, respectively, can be almost completely killed (Fig. 2). After lowing 5FU concentration to 100 *µ*g/ml and treating HepG2 cells with 100 *µ*g/ml 5FU in combination with antcin A in 5 *µ*g/ml, 10 *µ*g/ml, 15 *µ*g/ml, 20 *µ*g/ml and 25 *µ*g/ml, respectively, the cells can be almost completely killed (Fig. 3).

Furthermore, normal cell line CCD-966SK were treated with medium as control, 200 *µ*g/ml 5FU, 100 *µ*g/ml 5FU and 100 *µ*g/ml 5FU in combination with 25 *µ*g/ml antcin A, respectively, to test the effect of the combination of the invention on normal cells. As shown in Fig. 4, adding antcin A can increase cell viability of the cells treated with 5FU.

In addition, when HepG2 cells and CCD-966SK cells were treated with antcin A 10 *µ*g/ml in combination with 5FU in 12.5 *µ*g/ml, 25 *µ*g/ml, 50 *µ*g/ml, 100 *µ*g/ml and 200 -*µ*g/ml, respectively, the cancer cells (HepG2 cells) can be effectively inhibited, while the normal cells (CCD-966SK cells) have high viability (see Fig. 5). The results show that antcin A can enhance the effect of 5-FU in killing cancer cells (i.e., reduce the amount of 5-FU) while while keeping normal cells at high viability (i.e., reducing cytotoxicity to normal cells).

### Example 2 Cell Viability Assay (MTT assay) for Combination of antcin A and Tamoxifen

Except for 3.13 µg/ml tamoxifen+25µg/ml antcin A, 3.13 µg/ml 5FU+20µg/ml antcin A, 3.13 µg/ml tamoxifen +15µg/ml antcin A, 3.13 µg/ml tamoxifen +10µg/ml antcin A, 3.13 µg/ml tamoxifen +5µg/ml antcin A as testing samples, the control, testing conditions, materials and method are the same as Example 1. As shown in Fig. 6, the IC₅₀ of HepG2 cells treated with tamoxifen is 8.02 *µ*g/ml and the cells treated with 12.5 *µ*g/ml tamoxifen cannot be completely killed. By using low concentration of tamoxifen in combination with antcin A in 5 *µ*g/ml, 10 *µ*g/ml, 15 *µ*g/ml, 20 *µ*g/ml and 25 *µ*g/ml, respectively, the cell viabilities can be significantly reduced. As shown in Fig. 7, after treating HepG2 cells with 3.13 *µ*g/ml tamoxifen ("TAM") in combination with antcin A in 5 *µ*g/ml, 10 *µ*g/ml, 15 *µ*g/ml, 20 *µ*g/ml and 25 *µ*g/ml, respectively, the cell viabilities are 49.92%, 41.52%, 30.82%, 26.21% and 23.18%, respectively.

Furthermore, normal cell line CCD-966SK were treated with medium as control, 3.13 *µ*g/ml tamoxifen ("TAM") and 3.13 *µ*g/ml TAM in combination with 15 *µ*g/ml antcin A, respectively, to test the effect of the combination of the invention on normal cells. As shown in Fig. 8, adding antcin A can increase cell viability of the cells treated with TAM.

In addition, when HepG2 cells and CCD-966SK cells were treated with antcin A 10 *µ*g/ml in combination with TAM in 0.625 *µ*g/ml, 1.25 *µ*g/ml, 2.5 *µ*g/ml, 5 *µ*g/ml and 10 *µ*g/ml, respectively, the cancer cells (HepG2 cells) can be effectively inhibited, while the normal cells (CCD-966SK cells) have high viability (see Fig. 9). The results show that antcin A can enhance the effect of TAM in killing cancer cells (i.e., reduce the amount of 5-FU) while while keeping normal cells at high viability (i.e., reducing cytotoxicity to normal cells).

### Example 3 Cell Viability Assay (MTT assay) for Combinations of Antcin A and Sunitinib and antcin A and Mitoxantrone

The control, testing conditions, materials and method are the same as Example 1. As shown in Fig. 10, the IC₅₀ of HepG2 cells treated with sunitinib is about 25 *µ*g/ml and the normal cells (CCD-966SK cells) were also killed at this concentration. When HepG2 cells and CCD-966SK cells were treated with antcin A 10 *µ*g/ml in combination with sunitinib in 5 *µ*g/ml, 10 *µ*g/ml, 15 *µ*g/ml, 20 *µ*g/ml and 25 *µ*g/ml, respectively, the cancer cells (HepG2 cells) can be effectively inhibited, while the normal cells (CCD-966SK cells) have high viability (see Fig. 11). The results show that antcin A can enhance the effect of sunitinib in killing cancer cells (i.e., reduce the amount of 5-FU) while while keeping normal cells at higher viability (i.e., reducing cytotoxicity to normal cells).

Turning to mitoxantrone, as shown in Fig. 12, the IC₅₀ of HepG2 cells treated with mitoxantrone is about 2.5 *µ*g/ml and the viability of normal cells (CCD-966SK cells) was reduced at this concentration. When HepG2 cells and CCD-966SK cells were treated with antcin A 10 *µ*g/ml in combination with mitoxantrone in 2.5 *µ*g/ml, 5 *µ*g/ml, 6.25 *µ*g/ml, 10 *µ*g/ml and 12.5 *µ*g/ml, respectively, the cancer cells (HepG2 cells) can be effectively inhibited, while the normal cells (CCD-966SK cells) have higher viability (see Fig. 13). The results show that antcin A can enhance the effect of sunitinib in killing cancer cells (i.e., reduce the amount of 5-FU) while keeping normal cells at higher viability (i.e., reducing cytotoxicity to normal cells).

### Example 4 Animal Model Test for Combinations of the Invention, Aantcin A and 5FU, Actin A and Tamoxifen, Antcin A and sunitinib, and Antcin A and Mitoxantrone

2x10⁶ of HepG2 human liver cancer cells were injected to the back of NOD - SCID mice to form tumors. After the tumors grew to 10 x 10mm³, the combinations of the invention, antcin A at an amount of 1mg/kg and 5FU at the amounts of 20mg/kg, 10mg/kg, 5mg/kg, 2.5mg/kg, 1.25mg/kg, respectively, antcin A at an amount of 1mg/kg and tamoxifen at the amounts of 1mg/kg, 0.5mg/kg, 0.25mg/kg, 0.125mg/kg, 0.0625mg/kg, respectively, antcin A at an amount of 1mg/kg and sunitinib at the amounts of 2.5mg/kg, 2mg/kg, 1.5mg/kg, 1mg/kg, 0.5mg/kg, respectively, and antcin A at an amount of 1mg/kg and mitoxantrone at the amounts of 1.25mg/kg, 1mg/kg, 0.625mg/kg, 0.5mg/kg, 0.25mg/kg, respectively, were injected to abdominal cavity of the mice each day, respectively. Ethanol was injected to the mice as control. After one injection per day for 14 days, the mice were sacrificed and the size of the tumors were measured. The results show that the combinations of the invention significantly reduce the size of the tumors (about 20% to 70% reduction).

## Claims

1. A combination an anti-tumor drug in combination with antcin or a salt or ester derivative or a composition thereof, wherein the antcin or a salt or ester derivative or a composition thereof is in an amount to reduce an amount of the anti-tumor drug and provide low cytotoxity to normal cells; wherein the antcin has the following formula: 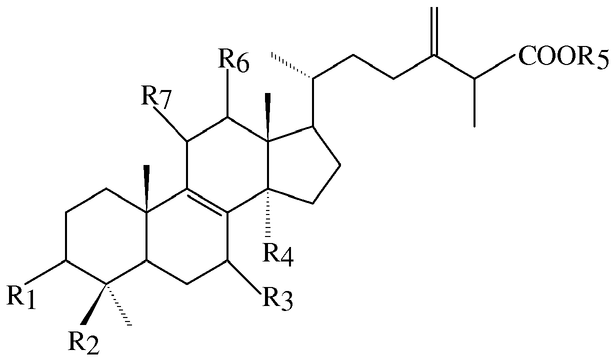 wherein
R₁ is =O or OH; R₂ is H or OH; R₃ is H, =O, OH or O-acetyl; R₄ is H or OH; R₅ is H; R₆ is H or OH; and R₇ is =O or OH.

2. The combination of Claim 1, wherein antcin is antcin A, antcin B, antcin C, antcin D, antcin E, antcin F, antcin H or antcin K, preferably antcin A.

3. The combination of Claim 1, wherein the salt of antcin includes base addition and acid addition salts.

4. The combination of Claim 1, wherein the ester derivative of antcin is alkyl actcin, alkynyl actcin, alkeny antcin.

5. The combination of Claim 1, wherein the ester derivative of antcin is methyl antcinate A, methyl antcinate B, methyl antcinate C, methyl antcinate G, methyl antcinate H, ethyl antcinate A or ethyl antcinate B.

6. The combination of Claim 1, wherein the composition of antcin is an extract of *Taiwanofungus camphoratus,* preferably the low polar fraction of *Taiwanofungus camphoratus* ethanol extract.

7. The combination of Claim 1, wherein the anti-tumor drug is an antimitotic drug, preferably taxol, paclitaxel, docetaxel, docetaxel, podophylotoxins, vincristine or vinblastine, an antimetabolite drug, preferably 5-fluorouracil (5FU), cytarabine, gemcitabine, pentostatin or methotrexate, an alkylating agent, preferably cis-platin, carboplatin, oxaliplatin or paraplidineatin, a nitrogen mustard, preferably nitrosourea, cyclophosphamide or ifosfamide, a drug which target DNA, preferably adriamycin, doxorubicin, pharmorubicin or epirubicin, a topoisomerase inhibitor, preferably camptothecin, etoposide, mitoxantrone or doxorubicin, a hormone antagonist, preferably tamoxifen, flutamide, leuprorelin, goserelin, cyproterone or octreotide, or a tyrosine kinase inhibitor, preferably imatinib, gefitinib, erlotinib or sunitinib.

8. The combination of Claim 7, wherein the antimitotic drug is the antimetabolite drug is the alkylating agent is the nitrogen mustard is the drugs which target DNA is the topoisomerase inhibitor is the hormone antagonist is the tyrosine kinase inhibitor is 11. The combination of Claim 9, wherein the antimitotic drug is paclitaxel, or docetaxel; the antimetabolite drug is 5FU; the alkylating agent is cis-platin or carboplatin; the nitrogen mustard is cyclophosphamide; the drug which target DNA is doxorubicin; the topoisomerases inhibitor is camptothecin, mitoxantrone or doxorubicin; the antiestrogen is tamoxifen; and the tyrosine kinase inhibitor is sunitinib.

9. The combination of Claim 7, wherein the anti-tumor agent is 5-fluorouracil, cytarabine, gemcitabine, purine analogues such as pentostatin, methotrexate, tamoxifen, cisplatin, carboplatin, oxaliplatin or paraplidineatin.

10. The combination of Claim 1, wherein the antcin or a salt or ester derivative or a composition thereof in combination with an antimetabolite drug or a hormone antagonist are in the amounts ranging from bout 0.5% (w/w) to about 95% (w/w) and about 99.5% (w/w) to about 5% (w/w) respectively.

11. A method of inhibiting and/or treating tumor with selectivity to tumors in a subject, which comprises administering to a subject an effective amount of the combination of Claim 1, wherein the combination comprises an anti-tumor drug in combination with antcin or a salt or ester derivative or a composition thereof and wherein the antcin or a salt or ester derivative or a composition thereof is in an amount to reduce an amount of the anti-tumor drug and provide low cytotoxity to normal cells..

12. The method of Claim 11, wherein the anti-tumor drug and the antcin, preferably antcin A, or a salt or ester derivative, preferably methyl antcinate A, methyl antcinate B, methyl antcinate C, methyl antcinate G, methyl antcinate H, ethyl antcinate A or ethyl antcinate B, or a composition thereof, preferably an extract of *Taiwanofungus camphoratus* containing antcin, are administered separately, concurrently or sequently.

13. The method of Claim 11, wherein the extract of *Taiwanofungus camphoratus* is the low polar fraction of *Taiwanofungus camphoratus* ethanol extract.

14. The method if Claim 11, wherein the anti-tumor drug is an antimitotic drug, an antimetabolite drug, an alkyling agent, a nitrogen mustard, a drug which target DNA, a topoisomerase inhibitor, a hormone antagonist or a tyrosine kinase inhibitor.

15. The method of Claim 11, wherein the tumor is selected from the group consisting of hepatocellular carcinoma, hepatoblastoma, colon carcinoma, pancreatic cancer, liver cancer, breast cancer, ovarian cancer,lung carcinoma, small cell lung carcinoma, bladder carcinoma, rectal carcinoma, head and neck cancer or brain cancer.
